# FASCICULE DE BREVET EUROPEEN

(11) **EP 2 959 863 B1**
(45) Date de publication et mention de la délivrance du brevet: **23.11.2016**
(21) Numéro de dépôt: 15171903.6
(22) Date de dépôt: 12.06.2015
(51) Int. Cl.: A61F 2/00, A61L 2/26, A61F 2/34

(54) **STRUCTURE DE CONDITIONNEMENT D'IMPLANTS COTYLOÏDIENS ET DES INSTRUMENTS DE POSE DE CES IMPLANTS**
VERPACKUNGSSTRUKTUR VON HÜFTGELENKSPFANNEN-IMPLANTATEN UND DEM ENTSPRECHENDEN OP-BESTECK ZUM EINSETZEN DIESER IMPLANTATE
PACKAGING STRUCTURE FOR COTYLOID IMPLANTS AND INSTRUMENTS FOR POSITIONING THE IMPLANTS

(30) Priorité: 25.06.2014 FR 1455940
(43) Date de publication de la demande: 30.12.2015
(73) Titulaire: Groupe Lepine, 69730 Genay (FR)
(72) Inventeur: Amorim, Cédric, 69580 Sathonay Camp (FR); Pfaifer, Patrick, 69250 Montany (FR)
(74) Mandataire: Jeannet, Olivier

(56) Documents cités:
- EP-A1- 1 393 695
- FR-A1- 2 797 180
- FR-A1- 2 906 146
- US-A1- 2007 034 538
- US-A1- 2013 226 183

## Description

La présente invention concerne une structure de conditionnement d'implants cotyloïdiens et des instruments de pose de ces implants.

Comment on le sait, un implant cotyloïdien est destiné à être mis en place dans la cavité cotyloïdienne d'un bassin afin de reconstituer une cavité articulaire, cette cavité articulaire étant destinée à recevoir une tête prothétique d'articulation portée par l'extrémité d'une tige fémorale, laquelle tige fémorale étant destinée à être implantée dans l'extrémité du fémur.

Chaque implant cotyloïdien comprend une cupule métallique destinée à être insérée étroitement dans la cavité cotyloïdienne et à être ancrée à l'os du bassin, et comprend également un noyau en un matériau favorisant le glissement, notamment en polyéthylène à haute densité, qui forme ladite cavité articulaire, ce noyau étant destiné à être reçu dans une cavité que forme la cupule.

La taille des cavités cotyloïdiennes étant très variable selon la taille, l'âge et le sexe d'un patient, un praticien doit disposer de séries d'implants cotyloïdiens de différentes tailles. Chaque implant d'une taille donnée est conditionné dans un emballage séparé.

Les instruments de pose de ces implants cotyloïdiens sont, quant à eux, selon une technique bien connue, regroupés sur un ou deux plateaux de présentation ; ils comprennent notamment :
- une série de râpes de différentes tailles, permettant la mise en forme de la cavité cotyloïdienne à appareiller ;
- une série de cupules d'essai de différentes tailles, permettant de tester l'adéquation de la cavité retaillée à la taille de l'implant cotyloïdien déterminée comme étant celle adaptée à la cavité cotyloïdienne à appareiller ;
- un manche apte à être assemblé à chaque râpe et à chaque cupule d'essai, pour la préhension de ces différentes râpes et cupules d'essai ;
- une série de platines d'impaction de différentes tailles, ayant des formes adaptées au montage sur elle des différentes tailles de cupules à implanter ;
- un manche d'impaction, apte à être assemblé à chaque platine d'impaction pour constituer un instrument permettant l'impaction d'une cupule dans la cavité cotyloïdienne à appareiller ;
- des noyaux d'essai de différentes tailles, adaptés aux cavités des différentes tailles de cupules ;
- un instrument d'insertion en force d'une tête fémorale prothétique de taille choisie dans le noyau de taille choisie.

La publication de demande de brevet N° FR 2 906 146, au nom de la demanderesse, illustre un plateau de présentation de tels instruments, conforme à cette technique.

Les platines d'impaction et le manche d'impaction sont notamment du type permettant une saisie de la cupule à implanter au moyen d'une dépression d'air, comme décrit par la publication de demande de brevet N° FR 2 797 180, également au nom de la demanderesse.

Ces plateaux de présentation, et les différents instruments qu'ils comportent, sont destinés à être stérilisés après chaque intervention chirurgicale, et sont donc réalisés en un matériau métallique apte à subir de nombreux cycles de stérilisation.

Un matériel de ce type a pour inconvénient d'être complexe et coûteux à fabriquer, compte tenu desdits matériaux, et d'être coûteux à utiliser, compte tenu desdites opérations de stérilisation. De plus, tout risque d'une stérilisation imparfaite du plateau et/ou des instruments, et donc d'une contamination d'un patient, n'est pas totalement écarté.

La présente invention a pour objectif de remédier à ces inconvénients essentiels.

La publication de demande de brevet N° US 2013/226183 A1 décrit des emballages comprenant, dans un premier compartiment, les éléments (cupule, noyau, vis) d'un implant d'une taille donnée, et, dans un deuxième compartiment relié au premier compartiment, les instruments correspondants. Tout ou partie de ces instruments est à usage unique.

La structure de conditionnement selon ce document antérieur ne permet pas de parfaitement atteindre l'objectif ci-dessus.

La structure de conditionnement à laquelle l'invention s'applique comprend des emballages d'implants contenant différentes tailles d'implants cotyloïdiens, et une structure de présentation des instruments de pose de ces implants cotyloïdiens, chaque emballage d'implant étant séparé des autres emballages d'implants et contenant la cupule et le noyau d'un implant d'une taille déterminée.

Selon l'invention, ladite structure de présentation comprend au moins deux emballages stériles d'instruments, physiquement séparés l'un de l'autre et physiquement séparés desdits emballages d'implants, qui sont dédiés chacun à une taille différente d'implant ;
un premier emballage d'instruments contient :
- une première cupule d'essai en un matériau peu onéreux tel qu'une matière plastique permettant que cette cupule d'essai soit à usage unique, cette première cupule d'essai ayant un diamètre équatorial externe déterminé, correspondant au diamètre équatorial externe de la cupule d'un implant cotyloïdien d'une première taille ;
- au moins un premier noyau d'essai en un matériau peu onéreux tel qu'une matière plastique permettant que ce noyau d'essai soit à usage unique ; chaque premier noyau d'essai présente une partie d'engagement dans la cavité formée par la cupule de première taille, et un embout de connexion à la tige fémorale à implanter ;
un deuxième emballage d'instruments contient :
- une deuxième cupule d'essai en un matériau peu onéreux tel qu'une matière plastique permettant que cette cupule soit à usage unique, cette deuxième cupule d'essai ayant un diamètre équatorial externe déterminé, correspondant au diamètre équatorial externe de la cupule d'un implant d'une deuxième taille ;
- au moins un deuxième noyau d'essai en un matériau peu onéreux tel qu'une matière plastique permettant que ce noyau d'essai soit à usage unique ; chaque deuxième noyau d'essai présente une partie d'engagement dans la cavité formée par la cupule de deuxième taille, et un embout de connexion à la tige fémorale à implanter.

Il sera compris que par "physiquement séparés", on entend que les emballages sont des unités distinctes, ayant des intégrités propres du point de vue de la stérilité, de telle sorte qu'un emballage puisse être transporté indépendamment d'un autre emballage, et puisse être ouvert sans que la stérilité d'un autre emballage soit affectée. Cette expression exclut que lesdits emballages puissent être des compartiments d'un même emballage unitaire.

L'invention repose ainsi sur la double idée consistant à prévoir en combinaison :
- une cupule d'essai et au moins un noyau d'essai à usage unique, et
- un emballage propre à ces instruments, dédié à une taille déterminée d'implant,
la structure de présentation des instruments comprenant plusieurs de ces emballages d'instruments, dont chacun est dédié à une taille déterminée d'implant, différente d'un emballage à l'autre.

L'utilisateur dispose donc d'une série d'emballages d'instruments séparés, dont chacun est dédié à une taille déterminée d'implant ; ainsi, pour implanter un implant cotyloïdien de taille X, il aura uniquement besoin d'ouvrir l'emballage contenant les instruments correspondant à cette taille X, et, pour implanter un implant cotyloïdien de taille Y, il aura uniquement besoin d'ouvrir l'emballage contenant les instruments correspondant à cette taille Y.

La structure de conditionnement selon l'invention permet par conséquent d'éliminer la nécessité de fabriquer des séries d'instruments de différentes tailles en des matériaux onéreux aptes à résister à de nombreux cycles de stérilisation ; elle permet également d'éliminer la nécessité d'opérer une stérilisation de l'ensemble des instruments de ces séries d'instruments, après chaque intervention chirurgicale, comme selon la technique antérieure. Elle permet en outre d'éliminer le risque d'une contamination résultant d'une stérilisation imparfaite.

De préférence, dans chaque emballage d'implant, la cupule de l'implant est assemblée à une platine d'impaction au moyen d'une dépression d'air opérée entre cette cupule et cette platine d'impaction.

La cupule de l'implant peut ainsi être manipulée et impactée au moyen de cette platine d'impaction, et aucune platine d'impaction n'a besoin d'être présente dans l'emballage d'instruments ni d'être assemblée à cette cupule en vue de la mise en place de cette cupule dans la cavité cotyloïdienne à appareiller. Une fois cette mise en place effectuée, une légère force exercée latéralement sur la platine d'impaction permet de décoller cette platine de la cupule, et donc de faire entrer de l'air entre cette platine et cette cupule, afin de désolidariser ces pièces l'une de l'autre.

De préférence, dans chaque emballage d'implant, le noyau contient la tête d'articulation prothétique insérée dans la cavité articulaire que ce noyau définit.

Cette insertion est ainsi opérée par le fabricant de la prothèse, avant conditionnement de la cupule et de l'ensemble noyau / tête dans ledit emballage d'implant. La nécessité de réaliser cette insertion au cours de l'intervention chirurgicale est ainsi éliminée, de même que, en conséquence, la nécessité d'avoir à disposer d'un instrument pour réaliser cette opération spécifique.

De préférence,
- chaque cupule d'essai et chaque platine d'impaction présentes dans les différents emballages formant la structure de conditionnement selon l'invention comprend une portion de connexion, et les portions de connexion de ces cupules d'essai et platines d'impaction sont identiques entre elles ; et
- la structure de conditionnement inclut au moins un manche de préhension comprenant un embout de connexion apte à venir en prise avec ces portions de connexion.

Un même manche est ainsi utilisable pour manipuler l'une quelconque des cupules d'essai et l'une quelconque des platines d'impaction, et un même type de manche peut être utilisé quelle que soit la taille de l'implant cotyloïdien implanté.

Chaque emballage d'instruments ou chaque emballage d'implant pourrait contenir un tel manche. De préférence, toutefois, la structure de conditionnement selon l'invention comprend plusieurs emballages de manches, contenant chacun un manche tel que précité, ces emballages de manches étant physiquement séparés desdits emballages d'instruments et desdits emballages d'implants.

Ainsi, il est simplement requis, lors de l'implantation d'un implant cotyloïdien d'une taille déterminée, d'ouvrir l'un desdits emballages stériles de manche.

Ce manche peut avoir une structure simple, notamment être formé par une pièce métallique unique, et donc être peu onéreux à fabriquer ; il est destiné à être réutilisé, et est donc en un matériau le rendant apte à être stérilisé, en particulier en métal.

De préférence, chaque emballage d'instruments contient plusieurs noyaux d'essai tels que précités, ayant des embouts de connexion de longueurs différentes.

Les différents noyaux d'essai d'un même emballage d'instruments peuvent ainsi être tour-à-tour mis en place sur l'embout d'une tige fémorale et être engagés dans la cavité de la cupule de l'implant afin de déterminer la longueur de col de la tige fémorale qui est la mieux adaptée à la restauration de l'articulation traitée.

Chaque emballage d'instruments peut notamment contenir trois noyaux d'essai, dont un à embout de connexion court, un à embout de connexion ayant une longueur moyenne et un à embout de connexion long.

De préférence,
- chaque implant cotyloïdien conditionné dans un emballage d'implant est de type dit "à double mobilité", dans lequel le noyau présente une forme extérieure sphérique et la cupule forme une cavité sphérique de réception de ce noyau avec possibilité mouvement multidirectionnel ; et
- chaque emballage d'instruments contient une pièce dite "réducteur", en un matériau peu onéreux tel qu'une matière plastique permettant que cette pièce soit à usage unique, ce réducteur comprenant une partie formant une cavité en forme de calotte sphérique creuse, permettant une prise d'appui contre le noyau de
l'implant cotyloïdien afin de permettre d'engager ce noyau dans la cavité formée par la cupule de cet implant.

Avantageusement, le réducteur comprend une portion de connexion permettant sa connexion audit manche.

Le manche permet également de saisir et manipuler ce réducteur.

De préférence, chaque emballage d'instruments contient une pièce dite "orienteur", apte à être montée sur ledit manche pour constituer un repère d'orientation angulaire de ce manche, et donc de la cupule de l'implant cotyloïdien, par rapport à la cavité cotyloïdienne à appareiller, au moment de réaliser l'impaction de cette cupule dans cette cavité.

L'invention sera bien comprise, et d'autres caractéristiques et avantages de celle-ci apparaîtront, en référence au dessin schématique annexé, représentant, à titre d'exemple non limitatif, une forme de réalisation préférée de divers instruments faisant partie de la structure de conditionnement concernée.
La figure 1 est une vue en perspective d'une cupule d'essai destinée à être contenue dans un emballage d'instruments faisant partie de cette structure de conditionnement ;
la figure 2 est une vue de cette cupule d'essai en plan, par son côté destiné à être relié à un manche préhension et de manipulation ;
la figure 3 est une vue en perspective d'un noyau d'essai destiné à être contenu dans ledit emballage d'instruments ;
la figure 4 est une vue de ce noyau d'essai de côté ;
la figure 5 est une vue en perspective d'une pièce dite "réducteur" destinée à être contenue dans ledit emballage d'instruments ;
la figure 6 est une vue en perspective d'un manche permettant la préhension et la manipulation de la cupule d'essai, de chaque noyau d'essai et de chaque réducteur ;
la figure 7 est une vue en perspective du même manche, équipé d'une pièce dite "orienteur" ; et
les figures 8 à 14 sont des vues en perspective des différents instruments précités, d'éléments d'une prothèse de hanche que ces instruments permettent d'implanter et d'une partie de l'os du bassin et du fémur appareillés.

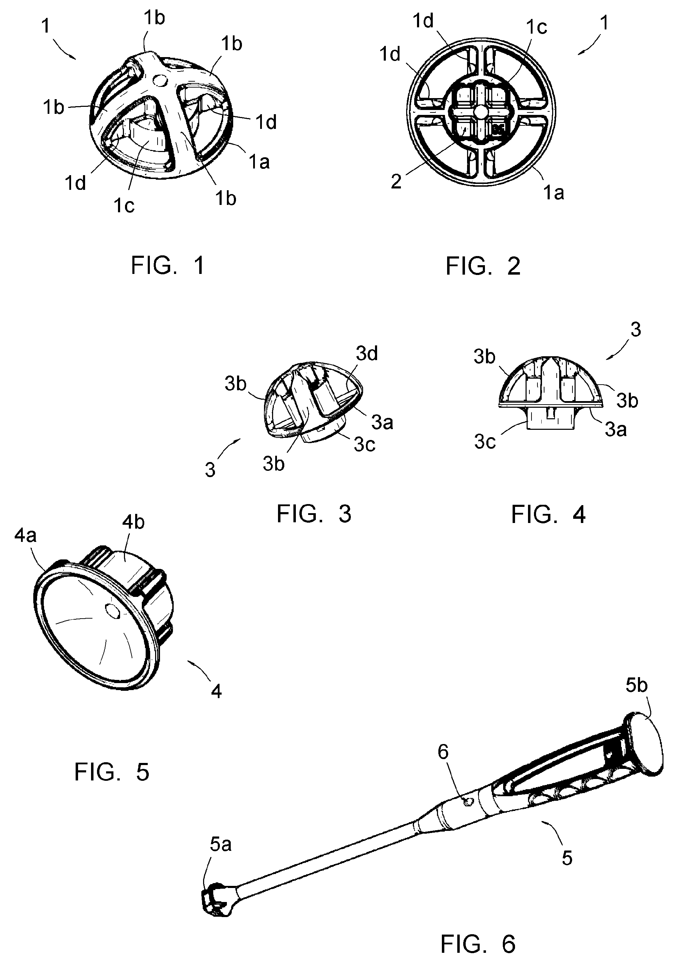

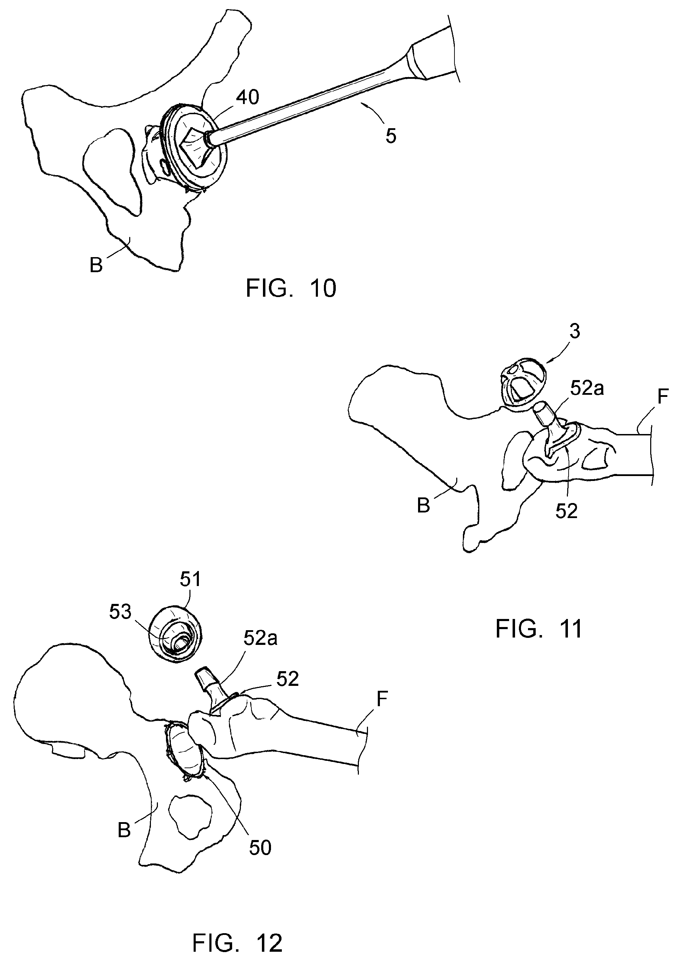

Les figures 1 et 2 représentent une cupule d'essai 1 destinée à être utilisée lors de la pose d'un implant cotyloïdien. Comme visible sur les figures 12 à 14, cet implant cotyloïdien est, dans l'exemple représenté, un implant du type bien connu dit "à double mobilité", comprenant :
- une cupule métallique 50 à face interne lisse et continue, définissant une cavité interne de forme sensiblement hémisphérique, et
- un noyau 51 en un matériau de glissement, notamment en polyéthylène à haute densité, présentant une forme extérieure sphérique et formant une cavité articulaire.

L'implant cotyloïdien est destiné à être mis en place dans la cavité cotyloïdienne d'un bassin B, et est associé à une tige fémorale 52, destinée à être implantée dans l'extrémité du fémur F. La tige 52 présente un col 52a sur lequel est destinée à être montée une tête prothétique 53, cette tête 53 étant en relation d'articulation avec le noyau 51 ; ce dernier est destiné à être reçu avec mobilité multidirectionnelle dans la cavité formée par la cupule 50.

Dans l'exemple représenté, et comme visible sur la figure 12, le noyau 51 contient, avant la mise en place de la prothèse, la tête 53 insérée dans la cavité articulaire qu'il définit. Cette insertion a été opérée par le fabricant de la prothèse, avant conditionnement de la cupule 50 et de l'ensemble noyau 51 / tête 53 dans un emballage stérile, dit ci-après "emballage d'implant".

La cupule d'essai 1 est en un matériau peu onéreux tel qu'une matière plastique permettant que cette cupule d'essai soit à usage unique. Elle présente une forme générale sensiblement hémisphérique, qui correspond à la forme extérieure de la cupule 50 ; en particulier, elle comprend un anneau équatorial 1a ayant un diamètre externe correspondant au diamètre équatorial externe de cette cupule 50 ; elle comprend également quatre branches courbes équidistantes 1 b se rejoignant en sa zone polaire, une structure centrale 1c formant une cavité 2 de connexion et quatre branches radiales 1d reliant cette structure centrale 1c à l'anneau 1a. La cavité 2 est dimensionnée de manière à recevoir avec frottement une extrémité de connexion 5a du manche 5, pour créer avec ce manche, une liaison permettant la préhension et la manipulation de la cupule d'assai 1 au moyen du manche, cette liaison étant toutefois réversible par action de séparation exercée manuellement.

Les figures 3 et 4 représentent un noyau d'essai 3 également destiné à être utilisé lors de la pose du même implant cotyloïdien, et également en un matériau peu onéreux tel qu'une matière plastique permettant que ce noyau d'essai soit à usage unique. Ce noyau d'essai 3 présente une structure similaire à celle de la cupule d'essai 1 : anneau équatorial 3a, branches courbes 3b, structure centrale 3c et branches radiales 3d de liaison de cette structure 3c à l'anneau 3a. L'anneau 3a et les branches 3b constituent une partie ayant une forme générale extérieure adaptée à un engagement ajusté du noyau d'essai 3 dans la cavité formée par la cupule 50, et la structure centrale 3c se prolonge au-delà de l'anneau 3a et définit une cavité légèrement conique de forme correspondant à celle d'un embout de montage que comprend la portion d'extrémité libre du col 52a. Cette structure centrale 3c forme ainsi un embout de connexion au col 52a de la tige fémorale 52.

Le noyau d'essai 3 fait partie d'une série de trois noyaux d'essai destinés à être contenu dans ledit emballage d'instruments, à savoir un premier noyau d'essai dans lequel ladite structure centrale 3c est courte, un deuxième noyau d'essai dans lequel ladite structure centrale 3c est de longueur moyenne, et un troisième noyau d'essai dans lequel ladite structure centrale 3c est longue. Ces trois noyaux d'essai sont destinés à être montés tour-à-tour sur l'embout précité du col 52a, alors que la tige fémorale n'est pas définitivement implantée, ou en utilisant une tige fémorale d'essai, de manière à permettre de déterminer, parmi une série de plusieurs tiges fémorales à cols de différentes longueurs, celle ayant le col dont la longueur est la plus appropriée à la restauration de l'articulation ; de la même manière, lorsque la tige fémorale comprend des cols interchangeables, ces noyaux d'essai permettent de déterminer le col interchangeable ayant la longueur la plus appropriée à cette même restauration.

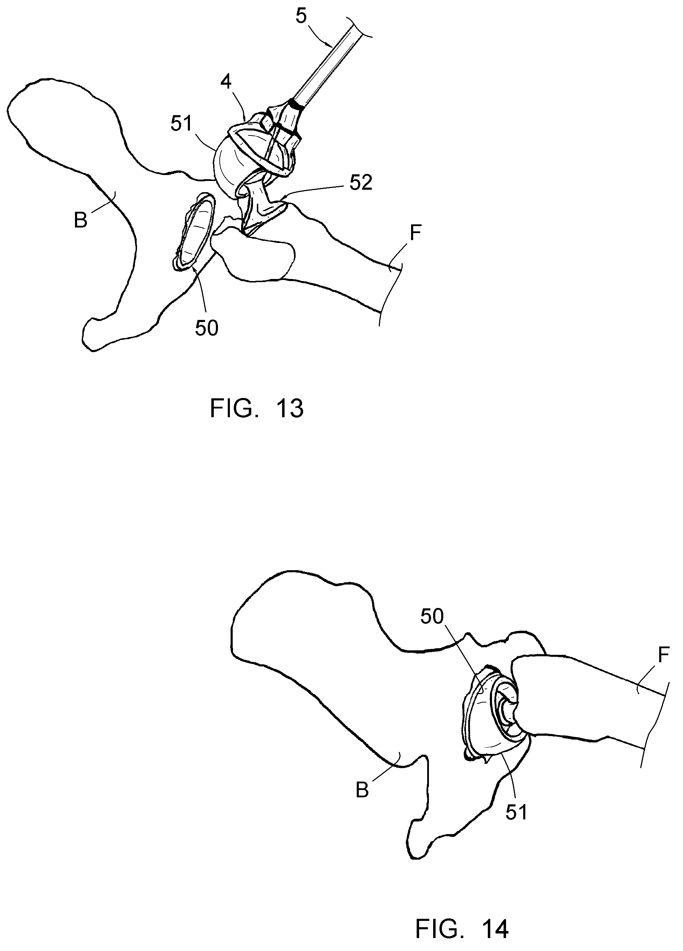

Le réducteur 4 est aussi en un matériau peu onéreux tel qu'une matière plastique permettant qu'il soit à usage unique. Il comprend une partie 4a formant une cavité en forme de calotte sphérique creuse, permettant, comme cela est visible sur la figure 13, une prise d'appui contre le noyau 51 pour permettre d'engager ce noyau dans la cavité formée par la cupule 50. Il comprend également une partie 4b, solidaire de la partie 4a, formant une cavité de connexion au manche 5, de sorte que ce manche 5 permet aussi de saisir et de manipuler ce réducteur 4.

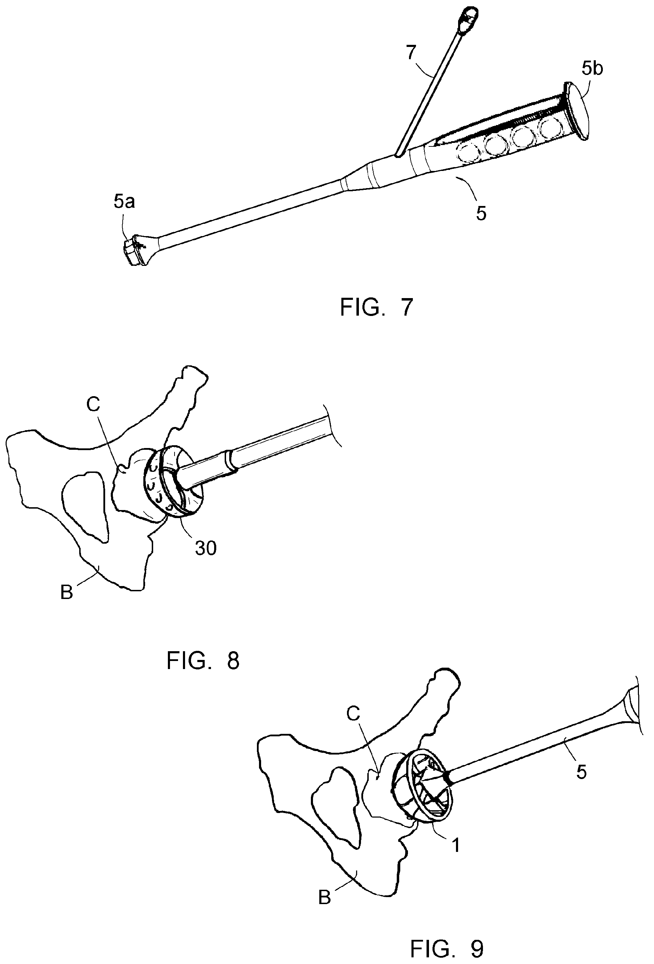

Le manche 5 est formé par une pièce métallique unique ; à l'opposé de l'embout de connexion 5a, il comprend une enclume 5b destinée à recevoir la frappe lors de l'impaction de la cupule 50. Il comprend également un trou 6 de réception de l'orienteur 7, comme visible sur la figure 7.

Cet orienteur 7 est aussi formé en un matériau peu onéreux tel qu'une matière plastique permettant qu'il soit à usage unique. Il comprend une tige se terminant par une portion d'extrémité libre aplatie ; une fois monté sur le manche 5, il constitue pour le praticien, avant de débuter l'impaction de la cupule 50, un repère d'orientation angulaire du manche 5, et donc de cette cupule 50, par rapport à la cavité cotyloïdienne à appareiller.

La structure de conditionnement selon l'invention comprend :
- une pluralité d'emballages d'implants qui contiennent différentes tailles d'implants cotyloïdiens, chaque emballage d'implant étant physiquement séparé des autres emballages d'implants et contenant un implant unique, d'une taille déterminée, à savoir la cupule 50 de cet implant et l'ensemble noyau 51 /têtue 53 ; chaque emballage d'implant comprend également une platine 40 d'impaction de la cupule 50, visible sur la figure 10, qui est reliée à cette cupule au moyen d'un vide d'air réalisé entre cette platine et cette cupule ;
- une pluralité d'emballages stériles d'instruments, physiquement séparés les uns des autres et physiquement séparés desdits emballages d'implants, qui contiennent chacun une cupule d'essai 1, trois noyaux d'essai 3, un réducteur 4 et un orienteur 7 tels que précités, la cupule d'essai 1 et les noyaux d'essai 3 d'un emballage ayant des dimensions adaptées à la cupule 50 d'un implant cotyloïdien d'une taille déterminée ; et
- une pluralité d'emballages stériles de manche, physiquement séparés les uns des autres et physiquement séparés desdits emballages d'implants et desdits emballages d'instruments, contenant chacun un manche 5.

En pratique, comme visible sur la figure 8, la cavité cotyloïdienne C à appareiller est tout d'abord fraisée au moyen d'une fraise 30 à usage unique ou stérilisable. Selon le diamètre de la fraise utilisée, le praticien détermine la taille de l'implant appropriée ; il ouvre alors l'emballage d'instruments correspondant à cette taille et un emballage de manche et, au moyen de la cupule d'assai 1 présente dans cet emballage d'instruments et du manche 5, il vérifie que la taille d'implant qu'il a déterminée est la bonne, cf. figure 9. Dans la négative, il peut ouvrir un autre emballage d'instruments contenant des instruments d'une taille supérieure ou inférieure selon le cas, sans que cela n'ait d'incidence économique trop importante, compte tenu du caractère peu onéreux des instruments 1, 3 et 4 ; dans l'affirmative, il ouvre l'emballage d'implant ayant la taille ainsi déterminée, saisit, au moyen du manche 5, l'ensemble platine d'impaction 40 / cupule 50 présent dans cet emballage, présente cet ensemble dans la cavité C, et, au moyen de l'orienteur 7 préalablement monté sur le manche 5, oriente convenablement le manche 5, et donc la cupule 50, par rapport à la cavité C puis réalise l'impaction, cf. figure 10.

Une fois l'impaction terminée, le praticien exerce une légère force latérale sur le manche 5 de manière à décoller la platine 40 de la cupule 50, et donc à faire entrer de l'air entre cette platine et cette cupule, afin de désolidariser ces pièces l'une de l'autre.

Le praticien monte ensuite tour-à-tour les différents noyaux d'essai 3 sur l'embout du col 52a de la tige fémorale 52 non définitivement implantée, ou d'une tige d'essai, pour déterminer la longueur de col la plus appropriée, comme décrit précédemment, cf. figure 11.

La tige fémorale 52 choisie est alors implantée puis l'ensemble noyau 51 / tête 53 est mis en place sur cette tige fémorale, cf. figure 12.

Le réducteur 4 est ensuite connecté au manche 5 pour opérer une réduction de l'articulation, c'est-à-dire pour engager le noyau 51 dans la cavité formée par la cupule 50, cf. figure 13, afin d'obtenir l'articulation prothétique, cf. figure 14.

Ainsi que cela apparaît de ce qui précède, l'invention fournit une structure de conditionnement d'implants cotyloïdiens de prothèses de hanche et de conditionnement des instruments de pose de ces implants, ayant notamment pour avantages déterminants de permettre :
- d'éliminer la nécessité de fabriquer des séries d'instruments de différentes tailles en des matériaux onéreux aptes à résister à de nombreux cycles de stérilisation ;
- d'éliminer la nécessité d'opérer une stérilisation de l'ensemble des instruments de ces séries d'instruments, après chaque intervention chirurgicale ;
- d'éliminer le risque d'une contamination résultant d'une stérilisation imparfaite.

L'invention a été décrite ci-dessus en référence à une forme de réalisation fournie à titre d'exemple. Il va de soi qu'elle n'est pas limitée à cette forme de réalisation et qu'elle s'étend à toutes les autres formes de réalisation couvertes par les revendications annexées.

## Revendications

1. Structure de conditionnement d'implants cotyloïdiens de prothèses de hanche et des instruments de pose de ces implants, comprenant des emballages d'implants qui contiennent différentes tailles d'implants cotyloïdiens et une structure de présentation des instruments de pose de ces implants cotyloïdiens, chaque emballage d'implant étant séparé des autres emballages d'implants et contenant la cupule (50) et le noyau (51) d'un implant d'une taille déterminée ;
ladite structure de présentation comprend au moins deux emballages stériles d'instruments, physiquement séparés l'un de l'autre et physiquement séparés desdits emballages d'implants, qui sont dédiés chacun à une taille différente d'implant ;
un premier emballage d'instruments contient :
- une première cupule d'essai (1) en un matériau peu onéreux tel qu'une matière plastique permettant que cette cupule d'essai soit à usage unique, cette première cupule d'essai (1) ayant un diamètre équatorial externe déterminé, correspondant au diamètre équatorial externe de la cupule (50) d'un implant cotyloïdien d'une première taille ;
- au moins un premier noyau d'essai (3) en un matériau peu onéreux tel qu'une matière plastique permettant que ce noyau d'essai (3) soit à usage unique ; chaque premier noyau d'essai (3) présente une partie d'engagement dans la cavité formée par la cupule (50) de première taille, et un embout de connexion à la tige fémorale (52) à implanter ;
un deuxième emballage d'instruments contient :
- une deuxième cupule d'essai (1) en un matériau peu onéreux tel qu'une matière plastique permettant que cette cupule soit à usage unique, cette deuxième cupule d'essai (1) ayant un diamètre équatorial externe déterminé, correspondant au diamètre équatorial externe de la cupule (50) d'un implant d'une deuxième taille ;
- au moins un deuxième noyau d'essai (3) en un matériau peu onéreux tel qu'une matière plastique permettant que ce noyau d'essai (3) soit à usage unique ; chaque deuxième noyau d'essai (3) présente une partie d'engagement dans la cavité formée par la cupule (50) de deuxième taille, et un embout de connexion à la tige fémorale (52) à implanter.

2. Structure de conditionnement selon la revendication 1, **caractérisée en ce que**, dans chaque emballage d'implant, la cupule (50) de l'implant est assemblée à une platine d'impaction (40) au moyen d'une dépression d'air opérée entre cette cupule (50) et cette platine d'impaction (40).

3. Structure de conditionnement selon la revendication 2, **caractérisée en ce que**, dans chaque emballage d'implant, le noyau (51) de l'implant contient la tête d'articulation prothétique (53) insérée dans la cavité articulaire que ce noyau (51) définit.

4. Structure de conditionnement selon la revendication 2 ou la revendication 3, **caractérisée en ce que** :
- chaque cupule d'essai (1) et chaque platine d'impaction (40) comprend une portion de connexion, et les portions de connexion de ces cupules d'essai et platines d'impaction sont identiques entre elles ; et
- la structure de conditionnement inclut au moins un manche (5) de préhension comprenant un embout de connexion (5a) apte à venir en prise avec ces portions de connexion.

5. Structure de conditionnement selon la revendication 4, **caractérisée en ce qu'**elle comprend plusieurs emballages de manches, contenant chacun un manche (5) tel que précité, ces emballages de manches étant physiquement séparés desdits emballages d'instruments et desdits emballages d'implants.

6. Structure de conditionnement selon l'une des revendications 1 à 5, **caractérisée en ce que** chaque emballage d'instruments contient plusieurs noyaux d'essai (3) ayant des embouts de connexion de longueurs différentes.

7. Structure de conditionnement selon l'une des revendications 1 à 6, **caractérisée en ce que** :
- chaque implant cotyloïdien conditionné dans un emballage d'implant est de type dit "à double mobilité", dans lequel le noyau (51) présente une forme extérieure sphérique et la cupule (50) forme une cavité sphérique de réception de ce noyau avec possibilité mouvement multidirectionnel ; et
- chaque emballage d'instruments contient une pièce (4) dite "réducteur", en un matériau peu onéreux tel qu'une matière plastique permettant que cette pièce soit à usage unique, ce réducteur (4) comprenant une partie formant une cavité en forme de calotte sphérique creuse, permettant une prise d'appui contre le noyau (51) de l'implant cotyloïdien afin de permettre d'engager ce noyau dans la cavité formée par la cupule (50) de cet implant.

8. Structure de conditionnement selon la revendication 7, **caractérisée en ce que** le réducteur (4) comprend une portion de connexion (4b) permettant sa connexion audit manche (5).

9. Structure de conditionnement selon l'une des revendications 4 à 8, **caractérisée en ce que** chaque emballage d'instruments contient une pièce (7) dite "orienteur", apte à être montée sur ledit manche (5) pour constituer un repère d'orientation angulaire de ce manche (5), et donc de la cupule (50) de l'implant cotyloïdien, par rapport à la cavité cotyloïdienne à appareiller, au moment de réaliser l'impaction de cette cupule dans cette cavité.

## Patentansprüche

1. Struktur zur Konditionierung von Cotyloidimplantaten von Hüftprothesen und von Instrumenten zur Platzierung dieser Implantate, umfassend Implantatverpackungen, die verschiedene Größen von Cotyloidimplantaten sowie eine Struktur zur Präsentation der Instrumente zur Platzierung dieser Cotyloidimplantate enthalten, wobei jede Implantatverpackung von den anderen Implantatverpackungen getrennt ist und die Pfanne (50) und den Kern (51) eines Implantats mit bestimmter Größe enthält;
wobei die Präsentationsstruktur wenigstens zwei sterile Instrumentenverpackungen umfasst, die physisch voneinander getrennt sind und physisch von den Implantatverpackungen getrennt sind, die jeweils einer anderen Implantatgröße zugeordnet sind;
wobei eine erste Instrumentenverpackung enthält:
- eine erste Testpfanne (1) aus einem wenig aufwändigen Material wie zum Beispiel einem Kunststoffmaterial, was es ermöglicht, dass diese Testpfanne zur einmaligen Verwendung ist, wobei diese erste Testpfanne (1) einen bestimmten äußeren Äquatorialdurchmesser hat, der dem äußeren Äquatorialdurchmesser der Pfanne (50) eines Cotyloidimplantats einer ersten Größe entspricht;
- wenigstens einen ersten Testkern (3) aus einem wenig aufwändigen Material wie zum Beispiel einem Kunststoffmaterial, was es ermöglicht, dass dieser Testkern (3) zur einmaligen Verwendung ist, wobei jeder erste Testkern (3) einen Teil zum Eingriff in den Hohlraum aufweist, der durch die Pfanne (50) der ersten Größe gebildet ist, sowie einen Ansatz zur Verbindung mit dem zu implantierenden Femurschaft (52);
wobei eine zweite Instrumentenverpackung enthält:
- eine zweite Testpfanne (1) aus einem wenig aufwändigen Material wie zum Beispiel einem Kunststoffmaterial, was es ermöglicht, dass diese Pfanne zur einmaligen Verwendung ist, wobei diese zweite Testpfanne (1) einen bestimmten äußeren Äquatorialdurchmesser hat, der dem äußeren Äquatorialdurchmesser der Pfanne (50) eines Implantats mit einer zweiten Größe entspricht;
- wenigstens einen zweiten Testkern (3) aus einem wenig aufwändigen Material wie zum Beispiel einem Kunststoffmaterial, was es ermöglicht, dass dieser Testkern (3) zur einmaligen Verwendung ist, wobei jeder zweite Testkern (3) einen Teil zum Eingriff in den Hohlraum aufweist, der durch die Pfanne (50) der zweiten Größe gebildet ist, sowie einen Ansatz zur Verbindung mit dem zu implantierenden Femurschaft (52).

2. Struktur zur Konditionierung nach Anspruch 1, **dadurch gekennzeichnet, dass** bei jeder Implantatverpackung die Pfanne (50) des Implantats mit einer Impaktionsplatte (40) mit Hilfe eines Luftunterdrucks zusammengefügt ist, der zwischen dieser Pfanne (50) und dieser Impaktionsplatte (40) vorgesehen ist.

3. Struktur zur Konditionierung nach Anspruch 2, **dadurch gekennzeichnet, dass** bei jeder Implantatverpackung der Kern (51) des Implantats den Prothesengelenkkopf (53) eingesetzt in den Gelenkhohlraum enthält, den dieser Kern (51) definiert.

4. Struktur zur Konditionierung nach Anspruch 2 oder Anspruch 3, **dadurch gekennzeichnet, dass**:
- jede Testpfanne (1) und jede Impaktionsplatte (40) einen Verbindungsbereich umfasst, und dass die Verbindungsbereiche dieser Testpfannen und Impaktionsplatten miteinander identisch sind; und
- die Struktur zur Konditionierung wenigstens eine Griffmanschette (5) enthält, die einen Verbindungsansatz (5a) umfasst, der dazu ausgelegt ist, in Eingriff mit diesen Verbindungsbereichen zu gelangen.

5. Struktur zur Konditionierung nach Anspruch 4, **dadurch gekennzeichnet, dass** sie mehrere Manschettenverpackungen umfasst, die jeweils eine Manschette (5) wie vorgenannt enthalten, wobei diese Manschettenverpackungen physisch von den Instrumentenverpackungen und den Implantatverpackungen getrennt sind.

6. Struktur zur Konditionierung nach einem der Ansprüche 1 bis 5, **dadurch gekennzeichnet, dass** jede Instrumentenverpackung mehrere Testkerne (3) enthält, die Verbindungsansätze mit unterschiedlichen Längen haben.

7. Struktur zur Konditionierung nach einem der Ansprüche 1 bis 6, **dadurch gekennzeichnet, dass**:a
- jedes Cotyloidimplantat, das in einer Implantatverpackung konditioniert ist, vom sogenannten Typ "mit doppelter Mobilität" ist, wobei der Kern (51) eine sphärische Außenform aufweist und die Pfanne (50) einen sphärischen Hohlraum zur Aufnahme dieses Kerns mit multidirektionaler Bewegungsmöglichkeit bildet; und
- jede Instrumentenverpackung ein Stück (4) enthält, das als "Reduzierer" bezeichnet wird, aus einem wenig aufwändigen Material wie zum Beispiel einem Kunststoffmaterial, was es ermöglicht, das dieses Stück zur einmaligen Verwendung ist, wobei dieser Reduzierer (4) einen Teil umfasst, der einen Hohlraum in Form einer sphärischen Hohlkalotte bildet, was einen Eingriff in Anlage gegen den Kern (51) des Cotyloidimplantats ermöglicht, um es zu ermöglichen, dass dieser Kern in dem Hohlraum angreift, der durch die Pfanne (50) dieses Implantats gebildet ist.

8. Struktur zur Konditionierung nach Anspruch 7, **dadurch gekennzeichnet, dass** der Reduzierer (4) einen Verbindungsbereich (4b) umfasst, der seine Verbindung mit der Manschette (5) ermöglicht.

9. Struktur zur Konditionierung nach einem der Ansprüche 4 bis 8, **dadurch gekennzeichnet, dass** jede Instrumentenverpackung ein Stück (7) enthält, das als "Orientierer" bezeichnet wird, das dazu ausgelegt ist, an der Manschette (5) montiert zu sein, um eine Markierung zur Winkelorientierung dieser Manschette (5) und somit der Pfanne (50) des Cotyloidimplantats bezüglich des hiermit zu kombinierenden Cotyloidhohlraums im Moment der Realisierung der Impaktion dieser Pfanne in diesen Hohlraum zu bilden.

## Claims

1. A packaging structure for cotyloïd implants of hip prosthesis and for instruments for putting these implants in place, including implant packs containing different sizes of cotyloïd implants and an exhibit structure for exposing these instruments for putting these cotyloïd implants in place, each implant pack being separated from the other implant packs and containing the cup (50) and the core (51) of an implant of a specified size;
said exhibit structure comprises at least two sterile instrument packs, physically separated from one another and physically separated from said implant packs, which are each dedicated to a different implant size;
a first package of instruments includes:
- a first test cup (1) made in an inexpensive material such as plastic, making it possible that this test cup is a single use cup, this first test cup (1) having a determined external equatorial diameter, corresponding to the external equatorial diameter of the cup (50) of a cotyloïd implant of a first size;
- at least a first test core (3) an inexpensive material such as plastic, making it possible that this test core (3) is a single use test core; each first test core (3) has an engaging portion for engaging into the cavity formed by the cup (50) of first size, and a connecting end for connection to the femoral stem (52) to be implanted;
a second instrument pack includes:
- a second test cup (1) in an inexpensive material such as a plastic material, making it possible that this test cup is a single use cup, this second test cup (1) having a determined external equatorial diameter, corresponding to the equatorial diameter external of the cup (50) of an implant of a second size;
- at least one second test core (3) in an inexpensive material such as plastic, making it possible that this test core (3) is a single use test core; each second test core (3) has an engaging portion for engaging into the cavity formed by the cup (50) of second size and a connecting end for connection to the femoral stem (52) to be implanted.

2. A package structure according to claim 1, **characterized in that**, in each implant pack, the cup (50) of the implant is assembled to an impaction plate (40) by means of an air depression drawn between this cup (50) and the impaction plate (40).

3. A package structure according to claim 2, **characterized in that**, in each implant pack, the core (51) of the implant contains the prosthetic articulation head (53) inserted into the articular cavity formed by that core (51).

4. A package structure according to claim 2 or claim 3, **characterized in that**:
- each test cup (1) and each impaction plate (40) comprise a connecting portion, and the connection portions of these test cups and impaction plates are identical to each other; and
- the packaging structure includes at least one gripping handle (5) comprising a connection end (5a) adapted to engage with these connecting portions.

5. package structure according to claim 4, **characterized in that** it comprises a plurality of handle packs, each containing a handle (5) as mentioned above, these handle packs being physically separated from said instrument packs and from said implant packs.

6. A packaging structure according to one of claims 1 to 5, **characterized in that** each instrument pack contains a plurality of test cores (3) having connection ends of different lengths.

7. A packaging structure according to one of claims 1 to 6, **characterized in that**:
- each cotyloid implant packed in an implant pack is a so-called "dual mobility" implant wherein the core (51) has a spherical outer shape and the cup (50) form a spherical cavity for receiving said core with a possibility multidirectional movement; and
- each instrument pack contains a so-called "reducing" part (4) made in an inexpensive material such as plastic making it possible that this part is a single use part, this part (4) comprising a portion forming a hollow spherical cap shaped cavity, making possible a bearing against the core (51) of the cotyloïd implant in order to allow the engagement of the core in the cavity formed by the cup (50) of the implant.

8. A packaging structure according to claim 7, **characterized in that** the part (4) comprises a connecting portion (4b) for its connection to said handle (5).

9. A packaging structure according to one of claims 4 to 8, **characterized in that** each instrument package contains a part (7) called "orienteer", adapted to be mounted on said handle (5) to constitute indicia for the angular orientation of the handle (5), and therefore of the cup (50) of the cotyloïd implant with respect to the cotyloïd cavity to be equipped, at the moment of carrying out the impaction of the cup in this cavity.
